# EUROPÄISCHE PATENTSCHRIFT

(11) **EP 0 954 277 B1**
(45) Veröffentlichungstag und Bekanntmachung des Hinweises auf die Patenterteilung: **13.12.2000**
(21) Anmeldenummer: 97914079.5
(22) Anmeldetag: 17.01.1997
(51) Int. Cl.: A61K 7/42

(54) **KOSMETISCHES SELBSTBRÄUNUNGSMITTEL MIT LICHTSCHUTZWIRKUNG**
COSMETIC SELF-TANNING AGENT HAVING A SUNSCREEN EFFECT
AGENT COSMETIQUE AUTOBRONZANT A EFFET DE PROTECTION SOLAIRE

(30) Priorität: 17.01.1996 DE 19603018
(43) Veröffentlichungstag der Anmeldung: 10.11.1999
(73) Patentinhaber: LANCASTER GROUP GmbH, 55116 Mainz (DE)
(72) Erfinder: MENZEL, Anette, Morris Plains, NJ 07950 (US); MACCHIO, Ralph, Flanders, NJ 07836 (US); STANZL, Klaus, White Plains, NJ 10605 (US); ZASTROW, Leonhard, MC-98000 Monte-Carlo (MC)
(74) Vertreter: Walter, Wolf-Jürgen
(86) Internationale Anmeldenummer: DE9700119
(87) Internationale Veröffentlichungsnummer: WO9725970

(56) Entgegenhaltungen:
- WO-A-92/17159
- WO-A-94/02176
- WO-A-94/23693

## Beschreibung

Die Erfindung betrifft ein kosmetisches Selbstbräunungsmittel auf Basis von Dihydroxyaceton mit Lichtschutzwirkung, mit welchem neben der Lichtschutzwirkung unterschiedliche Farbtöne auf der Haut erzielt werden können.

Die Verwendung von Dihydroxyaceton (DHA) als Bräunungsmittel für die menschliche Haut ist seit Jahren bekannt. Dabei ist DHA mit verschiedenen Begleitstoffen formuliert worden, um die normalerweise diesem Produkt innewohnenden Nachteile auszugleichen. DHA ist für eine etwas unnatürliche orangefarbene Hauttönung bekannt und weiterhin dafür, daß es sehr reaktionsfähig mit anderen Komponenten eines Gemisches ist, sich schnell zersetzt und einen für Kosmetika nicht akzeptablen Geruch aufweist. Außerdem dauert die Hauteinfärbung meist zu lange (mehrere Stunden) und verblaßt dann wieder zu schnell (nach Stunden bis wenigen Tagen).

Eine Kombination von Bräunungsmittel auf Basis von DHA und Lichtschutzmittel ist aus der WO 94/23693 bekannt. Dabei handelt es sich um eine O/W-Emulsion mit 0,1 bis 20 % DHA, 0,1 bis 30 % eines Sonnenschutzmittels, ausgewählt aus einer großen Gruppe dieser Mittel, und 0,1 - 10 % eines vernetzten kationischen Polymeren sowie 0,1 bis 10 % eines kationischen Emulgators.

Aus der EP-A-0627214 ist eine hautfärbende DHA-Formulierung bekannt, die zusätzlich einen hauthaftenden Farbstoff wie ein Eosin-derivat enthält.

Die US-A-4217344 beschreibt ein Lichtschutzmittel und Bräunungsmittel auf Basis von DHA, das zusätzlich Weinsäurealdehyd und eine spezielle Dispersion von Mikrokügelchen enthält.

Der vorliegende Erfindung liegt die Aufgabe zugrunde, ein in mehrfacher Hinsicht wirksames kosmetisches Selbstbräunungsmittel bereitzustellen. Mit Hilfe einer Kombination von Wirkstoffen soll eine mittlere lichtschützende Wirkung und zugleich eine selbstbräunende Wirkung erzielt werden, wobei die selbstbräunende Wirkung bereits nach kurzer Zeit eintritt und lange anhält und wobei durch unterschiedliche Zusammensetzung der Wirkstoffe mehrere Farbschattierungen für unterschiedliche Hauttypen ohne zusätzliche Farbkomponenten erreicht werden können.

Diese Aufgabe wird erfindungsgemäß gelöst mit einem kosmetisches Selbstbräunungsmittel mit Lichtschutzwirkung auf Basis von Dihydroxyaceton, das dadurch gekennzeichnet ist, daß es die folgenden Komponenten enthält, bezogen auf das Gesamtgewicht der Emulsion
(a) einen oder mehrere UV-Filter, ausgewählt aus der Gruppe, die aus Octylsalicylat, Octylmethoxycinnamat und Benzophenon-3 und deren Gemischen besteht, in einem Anteil von 2 bis 7,5 Gew-% der Einzelkomponenten und einem Anteil von 7 bis 18,5 Gew-% des Gemisches der Einzelkomponenten;
(b) Dihydroxyaceton (DHA) in einem liposomalen Transportsystem, bestehend aus DHA und Phospholipiden mit einem DHA-Anteil von 0,5 bis 5 Gew-%, wobei der Anteil der liposomalen Lösung im Bereich von 1,5 bis 25 Gew-% liegt und zusätzlich unverkapseltes DHA in einem Anteil von 1 bis 4 Gew-%;
(c) ein Antioxidationsmittel, ausgewählt aus der Gruppe, die aus Tocopherol, Ascorbinsäure, Calendula-Extrakt und einem Gemisch davon besteht, in einem Anteil von 0,01 bis 5,0 Gew-%;
(d) ein Feuchthaltemittel, ausgewählt aus der Gruppe, die aus Aloe vera, Phospholipiden, Natriumhyaluronat, Glycerin und Gemischen davon besteht, in einem Anteil der Feuchthaltemittel von 0,1 bis 16 Gew-%; und
üblichen kosmetischen Träger- und Hilfsstoffen. Die Angaben sind jeweils auf das Gewicht der Gesamtzusammensetzung bezogen.

Bevorzugt liegt das Anteil der UV-Filter (a) insgesamt im Bereich von 10 bis 16 Gew-%. Diese UV-Filter (a) liegen vorzugsweise in einem Gemisch von Octylsalicylat, Octylmethoxycinnamat und Benzophenon-3 vor, wobei das Verhältnis der Komponenten untereinander im Bereich von 1,0 bis 2,0 : 0,8 bis 2,5 : 0,8 bis 2,0 liegt. Ein besonders bevorzugtes Verhältnis ist 1:2:1.

Zusätzlich können aber auch als UV-Filter Melanin in löslicher Form (bis ca. 0,05 Gew-%) und Butyl-methoxydibenzoylmethan (bis 5 Gew-%) eingesetzt werden.

Weitere bevorzugte Bereiche der obigen Komponenten liegen für DHA (b) bei 0,8 bis 1,8 Gew-%, wobei der Anteil der liposomalen Lösung im Bereich von 1,5 bis 10,0 Gew-% liegt. Der Anteil der Antioxidationsmittel (c) liegt vorzugsweise im Bereich von 0,01 bis 0,5, und der Anteil der Feuchthaltemittel (d) liegt bevorzugt im Bereich von 0,7 bis 5 Gew-%.

Von den Feuchthaltemitteln sind in einer bevorzugten Ausführungsform Aloe vera und Phospholipide nebeneinander enthalten und wahlweise dann Glycerin oder Natriumhyaluronat.

Erfindungsgemäß wird eine selbstbräunende Wirkung in natürlich erscheinenden Farbtönen auf unterschiedlichen Hauttypen erreicht. Dabei setzt die Bräunung der Haut bereits nach etwa 30 bis 40 Minuten ein, hält bis zu 14 Tage an, und es tritt neben der lichtschützenden Wirkung zugleich auch ein deutlicher Feuchthalte-Effekt bei der Haut auf. Die Zusammensetzung penetriert leicht in die oberen Hautschichten bis zum Stratum corneum ein, so daß sich eine nur kurze Aufbringungszeit ergibt.

Der erreichte Lichtschutzfaktor liegt bei LSF 6 - 8.

Ein besonderer Vorteil der vorliegenden Erfindung besteht darin, daß sich wenigstens drei Grund-Farbschattierungen mit den Wirkstoffen der oben unter (a) bis (d) genannten Gruppen, jedoch in unterschiedlichen Zusammensetzungen, erzielen lassen.

Die drei Grund-Farbschattierungen sind:
1. ein heller (oder leuchtender) Goldfarbton (light golden glow), d.h. im Bereich von mehr gelblichem Braunton angesiedelt;
2. ein mittlerer bis dunkler Goldbronze-Farbton (medium/dark golden bronze), d.h. im Bereich eines dunkleren gelblichen Brauntones angesiedelt;
3. ein mittlerer bis dunkler rosiger Bronze-Farbton (medium/dark rosy bronze), d.h. im Bereich eines mehr rötlichen Brauntones angesiedelt.

Alle kosmetischen Zusammensetzungen der drei Grundschattierungen bzw -farbtöne sind weiterhin für die Haut nicht-reizend und hypoallergen, was einen besonderen Vorteil gegen-über anderen Produkten darstellt.

Zur Erreichung eines hellen Goldfarbtones enthält das Mittel folgende Komponenten:
(a) das Gemisch von Octylsalicylat, Octylmethoxycinnamat und Benzophenon-3 im Verhältnis untereinander von 1,0 bis 2,0 : 0,8 bis 2,5 : 0,8 bis 2,0, wobei der Anteil jedes einzelnen UV-Filters im Bereich von 3 bis 7,5 Gew-% liegt;
(b) DHA in einem liposomalen Trägersystem mit einem DHA-Anteil von 0,5 bis 1,0 Gew-% und zusätzlich unverkapseltes DHA in einem Anteil von 1,4 bis 2,6 Gew-%;
(c) ein Antioxidationsmittelgemisch mit einem Anteil von 0,15 bis 0,25 Gew-%;
(d) ein Feuchthaltemittelgemisch mit Glycerin mit einem Anteil von 2,5 bis 4 Gew-%;
und weitere kosmetische Hilfs- und Trägerstoffe.

Zur Erreichung eines mittleren bis dunklen Goldbronze-Farbtones enthält das Mittel folgende Komponenten:
(a) das Gemisch von Octylsalicylat, Octylmethoxycinnamat und Benzophenon-3 im Verhältnis untereinander von 1,0 bis 2,0 : 0,8 bis 2,5 : 0,8 bis 2,0, wobei der Anteil jedes einzelnen UV-Filters im Bereich von 3 bis 7,5 Gew-% liegt;
(b) DHA in einem liposomalen Trägersystem mit einem DHA-Anteil von 1,3 bis 1,7 Gew-% und zusätzlich unverkapseltes DHA in einem Anteil von 2,7 bis 3,8 Gew-%;
(c) ein Antioxidationsmittelgemisch mit einem Anteil von 0,15 bis 0,25 Gew-%;
(d) ein Feuchthaltemittelgemisch mit Glycerin mit einem Anteil von 2,7 bis 4 Gew-%;
und weitere kosmetische Hilfs- und Trägerstoffe.

Zur Erreichung eines mittleren bis dunklen rosigen Bronze-Farbtones enthält das Mittel folgende Komponenten:
(a) das Gemisch von Octylsalicylat, Octylmethoxycinnamat und Benzophenon-3 im Verhältnis untereinander von 1,0 bis 2,0 : 0,8 bis 2,5 : 0,8 bis 2,0, wobei der Anteil jedes einzelnen UV-Filters im Bereich von 3 bis 7,5 Gew-% liegt;
(b) DHA in einem liposomalen Trägersystem mit einem DHA-Anteil von 1,2 bis 1,6 Gew-% und zusätzlich unverkapseltes DHA in einem Anteil von 2,9 bis 4,0 Gew-%;
(c) ein Antioxidationsmittelgemisch mit einem Anteil von 0,05 bis 0,07 Gew-%;
(d) ein Feuchthaltemittelgemisch mit einem Anteil von 0,7 bis 1,1 Gew-%;
und weitere kosmetische Hilfs- und Trägerstoffe.

Ein besonders bevorzugtes Verhältnis von (a) ist bei allen drei Farbtönen 1:2:1.

Wie bereits oben ausgeführt, können neben den genannten UV-Absorptionsmitteln, die UV-A- und UV-B-strahlung absorbieren, auch lösliches Melanin und Butyl-methoxydibenzoylmethan vorhanden sein, die UV-A-Strahlung absorbieren.

Die erzielten Farbschattierungen werden durch Faktoren, die der Haut des jeweiligen Anwenders zuzuordnen sind, noch verstärkt oder vermindert, so daß die gewünschte Hautfärbung im wesentlichen mit den oben genannten drei Grund-Farbtönen erreicht werden kann.

Überraschenderweise wurde gefunden, daß die bekannten nachteiligen Wirkungen von DHA in der erfindungsgemäßen Kombination nicht oder in nur so geringem Umfang auftreten, daß sie nicht störend wirken. Insbesondere die bekannte Reaktionsfähigkeit von DHA, die zu einer Störung des Gleichgewichtes einer kompliziert zusammengesetzten kosmetischen Komposition führt und damit zur Instabilität des Systems, tritt hier nur stark vermindert auf. Es wird eine stabile Emulsion erhalten, sofern in den o.g. Grenzen der Anteile der einzelnen Bestandteile gearbeitet wird. Vorteilhaft wirkt sich die Verkapselung von DHA in Phospholipid-Liposomen aus, wodurch das natürliche Aussehen der Haut nach der Einfärbung verstärkt wird.

Ein weiterer überraschender Effekt besteht darin, daß mit der gegebenen kosmetischen Zusammensetzung unterschiedliche und natürlich wirkende Farbtöne nur durch Veränderung des Anteiles dieser Komponenten an der Gesamtzusammensetzung und entsprechende Anpassung der Restkomponenten erreicht und dabei stabile Emulsionen erhalten werden können.

Zu den üblichen kosmetischen Träger- und Hilfsstoffen, die weiterhin in der erfindungsgemäßen Zusammensetzung enthalten sein können, gehören Emulgatoren, verschiedene ölkomponenten (wie Fette, öle, Ester, Siliconöl usw.), weitere Stoffe wie Allantoin, Verdickungsmittel, Konservierungsmittel, Parfümöle, Komplexbildner, aktive Substanzen (wie z.B. Allantoin) und Wasser.

Die Erfindung soll nachstehend durch Beispiele näher erläutert werden, die jedoch keine Einschränkung der Erfindung darstellen. Die Prozentangaben sind stets auf das Gewicht bezogen, sofern nicht anderes angegeben ist.

### Beispiel 1

| Phase A | |
|---|---|
| Glyceryl Stearate/PEG-100 Stearate | 7 % |
| Sorbitan Stearate | 1 % |
| Palmitinsäure | 2 % |
| Stearinsäure | 2 % |
| Calendula-öl | 1 % |
| Octylpalmitat | 12 % |
| hydriertes Pflanzenöl | 2 % |
| Cyclomethicone | 1 % |
| Dimethicone | 2 % |
| Vitamingemisch C+E | 0,1 % |
| Octylsalicylat | 4 % |
| Benzophenon-3 | 7 % |
| Octylmethoxycinnamate | 4 % |

| Phase B | |
|---|---|
| deionisiertes Wasser | 28,2 % |
| Glycerin | 5 % |
| Xanthangummi | 0,2 % |

| Phase C | |
|---|---|
| Laureth-7/Polyacrylamid/C13-14 Isoparaffin | 1 % |

| Phase D | |
|---|---|
| Konservierungsmittel | 0,8 % |

| Phase E | |
|---|---|
| deionisiertes Wasser | 5 % |
| DHA | 3 % |
| Ethoxydiglycol | 7 % |

| Phase F | |
|---|---|
| deionisiertes Wasser | 1 % |
| Aloe vera Gel | 0,2 % |

| Phase G | |
|---|---|
| Parfüm | 0,5 % |

| Phase H | |
|---|---|
| DHA-Liposomen | 3 % |

Es wurden separat 8 Phasen hergestellt, die nacheinander miteinander vermischt wurden, teilweise unter intensiver Homogenisierung. Dabei wurden die Phasen A bis D bei erhöhter Temperatur (40 bis 70 °C) und die Phasen E bis H zum Schluß bei Umgebungstemperatur unter Homogenisierung eingearbeitet.

Man erhielt eine Creme mit feuchtigkeitspenden Eigenschaften, die einem LSF von 6 bis 8 hatte. Die Hautbräunung setzte etwa 40 Minuten nach dem Auftragen der Creme ein und erzielte einen mittleren bis dunklen Goldbronzeton. Die Bräunung hielt etwa 12 Tage an.

### Beispiel 2

| Phase A | |
|---|---|
| Stearath-2 und 21 | 4 % |
| Diethylenglycol+Dioctanoat/Diisononanoat | 2 % |
| C12-15 Alkyl Benzoate | 4 % |
| hydriertes Polyisobuten | 1 % |
| Calendula-öl | 1 % |
| Babassu-öl | 2 % |
| PPG-15 Stearyl ether | 2 % |
| Vitamingemisch A+E | 0,1 % |
| Octylsalicylat | 4 % |
| Benzophenon-3 | 3 % |
| Octylmethoxycinnamate | 7 % |

| Phase B | |
|---|---|
| deionisiertes Wasser | 32 % |
| Allantoin | 0,1 % |
| Na-Salz der Diethylentetraessigsäure | 0,1 % |
| Xanthangummi | 0,2 % |

| Phase C | |
|---|---|
| Laureth-7/Polyacrylamid/C13-14 Isoparaffin | 4 % |

| Phase D | |
|---|---|
| Konservierungsmittel | 0,5 % |

| Phase E | |
|---|---|
| Methyldibromo Glutaronitrile/Phenoxyethanol | 0,1 % |
| Cyclomethicone | 7 % |

| Phase F | |
|---|---|
| deionisiertes Wasser | 1 % |
| Aloe vera Gel | 0,2 % |

| Phase G | |
|---|---|
| Natriumhyaluronat | 0,2 % |
| deionisiertes Wasser | 1 % |

| Phase H | |
|---|---|
| deionisiertes Wasser | 5 % |
| DHA | 3,5 % |
| Ethoxydiglycol | 7 % |
| | |

| Phase I | |
|---|---|
| Parfüm | 0,5 % |

| Phase J | |
|---|---|
| DHA-Liposomen | 7,5 % |

Es wurden separat 10 Phasen hergestellt, die nacheinander miteinander vermischt wurden, teilweise unter intensiver Homogenisierung. Dabei wurden die Phasen A bis D bei erhöhter Temperatur (40 bis 70 °C) und die Phasen E bis J zum Schluß bei Umgebungstemperatur unter Homogenisierung eingearbeitet.

Man erhielt eine Creme mit feuchtigkeitspenden Eigenschaften, die einem LSF von 6 bis 8 hatte. Die Hautbräunung setzte etwa 30 bis 40 Minuten nach dem Auftragen der Creme ein und erzielte einen mittleren bis dunklen rosigen Bronzeton. Die Bräunung hielt etwa 10 bis 14 Tage an.

### Beispiel 3

| Phase A | |
|---|---|
| Glyceryl Stearate/PEG-100 Stearate | 7 % |
| Sorbitan Stearate | 1 % |
| Palmitinsäure | 2 % |
| Stearinsäure | 1 % |
| Calendula-öl | 1 % |
| Octylpalmitat | 5 % |
| hydriertes Pflanzenöl | 8 % |
| Cyclomethicone | 2 % |
| Dimethicone | 1 % |
| Vitamingemisch A+E | 0,1 % |
| Octylsalicylat | 4 % |
| Benzophenon-3 | 3 % |
| Octylmethoxycinnamate | 7 % |

| Phase B | |
|---|---|
| deionisiertes Wasser | 40,2 % |
| Glycerin | 4 % |
| Xanthangummi | 0,2 % |

| Phase C | |
|---|---|
| Laureth-7 /Polyacrylamid/C13-14 Isoparaffin | 1 % |

| Phase D | |
|---|---|
| Konservierungsmittel | 0,8 % |

| Phase E | |
|---|---|
| deionisiertes Wasser | 5 % |
| DHA | 2 % |

| Phase F | |
|---|---|
| deionisiertes Wasser | 1 % |
| Aloe vera Gel | 0,2 % |

| Phase G | |
|---|---|
| Parfüm | 0,5 % |

| Phase H | |
|---|---|
| DHA-Liposomen | 2 % |

Es wurde wie im Beispiel 1 gearbeitet.

Man erhielt eine Creme mit feuchtigkeitspenden Eigenschaften, die einem LSF von 6 bis 8 hatte. Die Hautbräunung setzte etwa 30 bis 40 Minuten nach dem Auftragen der Creme ein und erzielte einen hell leuchtenden Goldfarbton. Die Bräunung hielt etwa 10 bis 14 Tage an.

## Patentansprüche

1. Kosmetisches Selbstbräunungsmittel mit Lichtschutzwirkung auf Basis von Dihydroxyaceton, dadurch gekennzeichnet, daß es die folgenden Komponenten enthält, bezogen auf das Gesamtgewicht der Emulsion
(a) 7 bis 18,5 Gew-% eines Gemisches der UV-Filter Octylsalicylat, Octylmethoxycinnamat und Benzophenon-3 in einem Verhältnis untereinander von 1,0 bis 2,0 : 0,8 bis 2,5 : 0,8 bis 2,0;
(b) Dihydroxyaceton (DHA) in einem liposomalen Transportsystem, bestehend aus DHA und Phospholipiden mit einem DHA-Anteil von 0,5 bis 5 Gew-%, wobei der Anteil der liposomalen Lösung im Bereich von 1,5 bis 25 Gew-% liegt und zusätzlich unverkapseltes DHA in einem Anteil von 1 bis 4 Gew-%, jeweils bezogen auf das Gesamtgemisch;
c) ein Antioxidationsmittel, ausgewählt aus der Gruppe, die aus Tocopherol, Ascorbinsäure, Calendula-Extrakt und einem Gemisch davon besteht, in einem Anteil von 0,01 bis 5,0 Gew-% vom Gesamtgemisch;
d) ein Feuchthaltemittel, ausgewählt aus der Gruppe die aus Aloe vera, Phospholipiden, Phospholipidgemischen, Natriumhyaluronat, Glycerin und Gemischen davon besteht, in einem Anteil der Feuchthaltemittel von 0,1 bis 16 Gew-% vom Gesamtgemisch; und kosmetische Träger- und Hilfsstoffe.

2. Mittel nach Anspruch 1, dadurch gekennzeichnet, daß der Anteil der UV-Filter (a) insgesamt im Bereich von 10 bis 16 Gew-% liegt.

3. Mittel nach Anspruch 1, dadurch gekennzeichnet, daß der Anteil an DHA (b) im Bereich von 0,8 bis 1,8 Gew-% liegt, wobei der Anteil der liposomalen Lösung im Bereich von 1,5 bis 10,0 Gew-% liegt.

4. Mittel nach Anspruch 1, dadurch gekennzeichnet, daß der Anteil der Antioxidationsmittel (c) im Bereich von 0,01 bis 0,5 liegt.

5. Mittel nach Anspruch 1, dadurch gekennzeichnet, daß der Anteil der Feuchthaltemittel (d) im Bereich von 0,7 bis 5 Gew-% liegt.

6. Mittel nach einem der Ansprüche 1 bis 5, dadurch gekennzeichnet, daß es zur Erreichung eines hellen Goldfarbtones folgende Komponenten enthält
(a) das Gemisch von Octylsalicylat, Octylmethoxycinnamat und Benzophenon-3 im Verhältnis untereinander von 1,0 bis 2,0 : 0,8 bis 2,5 : 0,8 bis 2,0, wobei der Anteil jedes einzelnen UV-Filters im Bereich von 3 bis 7,5 Gew-% liegt;
(b) DHA in einem liposomalen Trägersystem mit einem DHA-Anteil von 0,5 bis 1,0 Gew-% und zusätzlich unverkapseltes DHA in einem Anteil von 1,4 bis 2,6 Gew-%;
(c) ein Antioxidationsmittelgemisch aus Tocopherol, Ascorbinsäure und Calendula-Extrakt mit einem Anteil von 0,15 bis 0,25 Gew-%;
(d) ein Feuchthaltemittelgemisch mit Glycerin mit einem Anteil von 2,5 bis 4 Gew-%;
und weitere kosmetische Hilfs- und Trägerstoffe.

7. Mittel nach einem der Ansprüche 1 bis 5, dadurch gekennzeichnet, daß es zur Erreichung eines mittleren bis dunklen Goldfarbtones folgende Komponenten enthält
(a) das Gemisch von Octylsalicylat, Octylmethoxycinnamat und Benzophenon-3 im Verhältnis untereinander von 1,0 bis 2,0 : 0,8 bis 2,5 : 0,8 bis 2,0, wobei der Anteil jedes einzelnen UV-Filters im Bereich von 3 bis 7,5 Gew-% liegt;
(b) DHA in einem liposomalen Trägersystem mit einem DHA-Anteil von 1,3 bis 1,7 Gew-% und zusätzlich unverkapseltes DHA in einem Anteil von 2,7 bis 3,8 Gew-%;
(c) ein Antioxidationsmittelgemisch aus Tocopherol, Ascorbinsäure und Calendula-Extrakt mit einem Anteil von 0,15 bis 0,25 Gew-%;
(d) ein Feuchthaltemittelgemisch mit Glycerin mit einem Anteil von 2,7 bis 4 Gew-%;
und weitere kosmetische Hilfs- und Trägerstoffe.

8. Mittel nach einem der Ansprüche 1 bis 5, dadurch gekennzeichnet, daß es zur Erreichung eines mittleren bis dunklen rosigen Farbtones folgende Komponenten enthält
(a) das Gemisch von Octylsalicylat, Octylmethoxycinnamat und Benzophenon-3 im Verhältnis untereinander von 1,0 bis 2,0 : 0,8 bis 2,5 : 0,8 bis 2,0, wobei der Anteil jedes einzelnen UV-Filters im Bereich von 3 bis 7,5 Gew-% liegt;
(b) DHA in einem liposomalen Trägersystem mit einem DHA-Anteil von 1,2 bis 1,6 Gew-% und zusätzlich unverkapseltes DHA in einem Anteil von 2,9 bis 4,0 Gew-%;
(c) ein Antioxidationsmittelgemisch aus Tocopherol, Ascorbinsäure und Calendula-Extrakt mit einem Anteil von 0,02 bis 0,04 Gew-%;
(d) ein Feuchthaltemittel mit einem Anteil von 0,7 bis 1,1 Gew-%;
und weitere kosmetische Hilfs- und Trägerstoffe.

9. Mittel nach einem der Ansprüche 1, 6, 7 oder 8, dadurch gekennzeichnet, daß der Anteil der UV-Filter (a) ein Gemisch von Octylsalicylat, Octylmethoxycinnamat und Benzophenon-3 ist in einem Verhältnis untereinander von 1:2:1.

## Claims

1. A cosmetic self-tanning agent with a sunscreen effect based on dihydroxyacetone, comprising the following ingredients, based on the total weight of the emulsion:
(a) 7 to 18.5 percent by weight of a mixture of the UV filters octyl salicylate, octyl methoxycinnamate and benzophenone-3 in a ratio of 1.0-2.0:0.8-2.5:0.8-2.0;
(b) dihydroxyacetone (DHA) in a liposomal transport system consisting of DHA and phospholipids with a DHA content of 0.5 to 5 percent by weight, where the amount of liposomal solution is in the range of 1.5 to 25 percent by weight, plus also unencapsulated DHA in an amount of 1 to 4 percent by weight, each based on the total weight of the mixture;
(c) an antioxidant selected from the group consisting of tocopherol, ascorbic acid, calendula extract and a mixture thereof, in an amount of 0.01 to 5.0 percent by weight of the total mixture;
(d) a moisturizer selected from the group consisting of aloe vera, phospholipids, phospholipid mixtures, sodium hyaluronate, glycerol and mixtures thereof, in an amount of 0.1 to 16 percent by weight of the total mixture;
and cosmetic vehicles and additives.

2. An agent according to Claim 1, wherein the total amount of the UV filters (a) is in the range of 10 to 16 percent by weight.

3. An agent according to Claim 1, wherein the amount of DHA (b) is in the range of 0.8 to 1.8 percent by weight, where the amount of the liposomal solution is in the range of 1.5 to 10.0 percent by weight.

4. An agent according to Claim 1, wherein the amount of antioxidants (c) is in the range of 0.01 to 0.5 percent by weight.

5. An agent according to Claim 1, wherein the amount of moisturizers (d) is in the range of 0.7 to 5 percent by weight.

6. An agent according to one of Claims 1 through 5, wherein it contains the following ingredients to achieve a light golden color:
(a) the mixture of octyl salicylate, octyl methoxycinnamate and benzophenone-3 in a ratio of 1.0-2.0:0.8-2.5:0.8-2.0, with the amount of each individual UV filter being in the range of 3 to 7.5 percent by weight;
(b) DHA in a liposomal carrier system with a DHA content of 0.5 to 1.0 percent by weight, plus unencapsulated DHA in an amount of 1.4 to 2.6 percent by weight;
(c) an antioxidant mixture of tocopherol, ascorbic acid and calendula extract in an amount of 0.15 to 0.25 percent by weight;
(d) a moisturizer mixture with glycerol in an amount of 2.5 to 4 percent by weight;
plus other cosmetic vehicles and additives.

7. An agent according to one of Claims 1 through 5, wherein it contains the following ingredients to achieve a medium to dark bronze color:
(a) the mixture of octyl salicylate, octyl methoxycinnamate and benzophenone-3 in a ratio of 1.0-2.0:0.8-2.5:0.8-2.0, where the amount of each individual UV filter is in the range of 3 to 7.5 percent by weight;
(b) DHA in a liposomal carrier system with a DHA content of 1.3 to 1.7 percent by weight, plus unencapsulated DHA in an amount of 2.7 to 3.8 percent by weight;
(c) an antioxidant mixture of tocopherol, ascorbic acid and calendula extract in an amount of 0.15 to 0.25 percent by weight;
(d) a moisturizer mixture with glycerol in an amount of 2.7 to 4 percent by weight;
and other cosmetic vehicles and additives.

8. An agent according to one of Claims 1 through 5, wherein it contains the following ingredients to achieve a medium to dark rosy bronze color:
(a) the mixture of octyl salicylate, octyl methoxycinnamate and benzophenone-3 in a ratio of 1.0-2.0:0.8-2.5:0.8-2.0, where the amount of each individual Uv filter is in the range of 3 to 7.5 percent by weight;
(b) DHA in a liposomal carrier system with a DHA content of 1.2 to 1.6 percent by weight, plus additional unencapsulated DHA in an amount of 2.9 to 4.0 percent by weight;
(c) an antioxidant mixture of tocopherol, ascorbic acid and calendula extract in an amount of 0.02 to 0.04 percent by weight;
(d) a moisturizer mixture in an amount of 0.7 to 1.1 percent by weight;
plus other cosmetic vehicles and additives.

9. An agent according to one of Claims 1, 6, 7 or 8, wherein the UV filters (a) comprise a mixture of octyl salicylate, octyl methoxycinnamate and benzophenone-3 in a ratio of 1:2:1.

## Revendications

1. Agent cosmétique autobronzant à effet de protection solaire à base de dihydroxyacétone, caractérisé en ce qu'il contient les composants suivants, par rapport au poids total de l'émulsion :
(a) 7 à 18,5 % en poids d'un mélange des filtres UV salicylate d'octyle, méthoxycinnamate d'octyle et benzophénone-3, dans un rapport mutuel de 1,0 à 2,0 : 0,8 à 2,5 : 0,8 à 2,0;
(b) de la dihydroxyacétone (DHA) dans un système de transport liposomal constitué de DHA et de phospholipides avec une fraction de DHA de 0,5 à 5 % en poids, la proportion de la solution liposomale se situant dans l'intervalle de 1,5 à 25 % en poids, plus de la DHA non-encapsulée en une proportion de 1 à 4 % en poids, toujours rapportée au mélange global;
(c) un antioxydant sélectionné dans le groupe comprenant le tocophérol, l'acide ascorbique, la calenduline et un mélange de ceux-ci, en une proportion de 0,01 à 5,0 % en poids du mélange global;
(d) un agent de rétention d'humidité, sélectionné dans le groupe comprenant l'aloe vera, des phospholipides, des mélanges de phospholipides, l'hyaluronate de sodium, le glycérol et des mélanges de ceux-ci, avec une proportion des agents de rétention d'humidité de 0,1 à 16 % en poids du mélange global; et
des substances de support et adjuvants cosmétiques.

2. Agent selon la revendication 1, caractérisé en ce que la proportion des filtres UV (a) se situe, dans l'ensemble, dans l'intervalle de 10 à 16 % en poids.

3. Agent selon la revendication 1, caractérisé en ce que la proportion de DHA (b) se situe dans l'intervalle de 0,8 à 1,8 % en poids, la proportion de la solution liposomale se situant dans l'intervalle de 1,5 à 10 % en poids.

4. Agent selon la revendication 1, caractérisé en ce que la proportion des antioxydants (c) se situe dans l'intervalle de 0,01 à 0,5.

5. Agent selon la revendication 1, caractérisé en ce que la proportion des agents de rétention d'humidité (d) se situe dans l'intervalle de 0,7 à 5 % en poids.

6. Agent selon l'une des revendications 1 à 5, caractérisé en ce que pour aboutir à une teinte dorée claire, il contient les composants suivants :
(a) le mélange de salicylate d'octyle, de méthoxycinnamate d'octyle et de benzophénone-3, dans un rapport mutuel de 1,0 à 2,0 : 0,8 à 2,5 : 0,8 à 2,0, la proportion de chaque filtre UV se situant dans l'intervalle de 3 à 7,5 % en poids;
(b) de la DHA dans un système de transport liposomal, avec une fraction de DHA de 0,5 à 1,0 % en poids, plus de la DHA non-encapsulée en une proportion de 1,4 à 2,6 % en poids;
(c) un mélange d'antioxydants comprenant du tocophérol, de l'acide ascorbique et de la calenduline en une proportion de 0,15 à 0,25 % en poids;
(d) un mélange d'agents de rétention d'humidité au glycérol, en une proportion de 2,5 à 4 % en poids; et d'autres substances de support et adjuvants cosmétiques.

7. Agent selon l'une des revendications 1 à 5, caractérisé en ce que pour aboutir à une teinte dorée moyenne à foncée, il contient les composants suivants :
(a) le mélange de salicylate d'octyle, de méthoxycinnamate d'octyle et de benzophénone-3, dans un rapport mutuel de 1,0 à 2,0 : 0,8 à 2,5 : 0,8 à 2,0, la proportion de chaque filtre UV se situant dans l'intervalle de 3 à 7,5 % en poids;
(b) de la DHA dans un système de transport liposomal, avec une fraction de DHA de 1,3 à 1,7 % en poids, plus de la DHA non-encapsulée en une proportion de 2,7 à 3,8 % en poids;
(c) un mélange d'antioxydants comprenant du tocophérol, de l'acide ascorbique et de la calenduline en une proportion de 0,15 à 0,25 % en poids;
(d) un mélange d'agents de rétention d'humidité au glycérol, en une proportion de 2,7 à 4 % en poids;
et d'autres substances de support et adjuvants cosmétiques.

8. Agent selon l'une des revendications 1 à 5, caractérisé en ce que pour aboutir à une teinte rosée moyenne à foncée, il contient les composants suivants :
(a) le mélange de salicylate d'octyle, de méthoxycinnamate d'octyle et de benzophénone-3, dans un rapport mutuel de 1,0 à 2,0 : 0,8 à 2,5 : 0,8 à 2,0, la proportion de chaque filtre UV se situant dans l'intervalle de 3 à 7,5 % en poids;
(b) de la DHA dans un système de transport liposomal, avec une fraction de DHA de 1,2 à 1,6 % en poids, plus de la DHA non-encapsulée en une proportion de 2,9 à 4,0 % en poids;
(c) un mélange d'antioxydants comprenant du tocophérol, de l'acide ascorbique et de la calenduline en une proportion de 0,02 à 0,04 % en poids;
(d) un mélange d'agents de rétention d'humidité au glycérol, en une proportion de 0,7 à 1,1 % en poids;
et d'autres substances de support et adjuvants cosmétiques.

9. Agent selon l'une des revendications 1, 6, 7 ou 8, caractérisé en ce que la fraction des filtres UV (a) est un mélange de salicylate d'octyle, de méthoxycinnamate d'octyle et de benzophénone-3, dans un rapport mutuel de 1:2:1.
